Europäisches Patentamt

European Patent Office

Office européen des brevets

(11) Numéro de publication: **0 408 440 A1**

# DEMANDE DE BREVET EUROPEEN

(21) Numéro de dépôt: 90401980.9

(22) Date de dépôt: 09.07.90

(51) Int. Cl.⁵: **A61K 31/485**, A61K 31/505, A61K 31/557, //(A61K31/485, 31:415,31:505),(A61K31/557, 31:505,31:485,31:475,31:415)

(30) Priorité: 11.07.89 FR 8909332

(43) Date de publication de la demande:
16.01.91 Bulletin 91/03

(84) Etats contractants désignés:
BE CH DE GB IT LI SE

(71) Demandeur: **Virag, Ronald**
**5, avenue Clodoald**
**F-92210 Saint-Cloud(FR)**

(72) Inventeur: **Virag, Ronald**
**5, avenue Clodoald**
**F-92210 Saint-Cloud(FR)**

(74) Mandataire: **Simonnot, Bernard et al**
**Cabinet Simonnot 35 rue de Clichy**
**F-75442 Paris Cédex 09(FR)**

(54) **Médicament vaso-actif.**

㊗ L'invention a trait au domaine des médicaments vaso-actifs.

Le médicament vaso-actif, du type constitué par une association de principes actifs à la papavérine, est caractérisé par le fait qu'il comprend au moins un alpha-bloquant et un inhibiteur de la phosphodiastérase, représentant au total 5 à 20 % en poids par rapport à la papavérine.

Application au traitement de l'impuissance chez l'homme.

# LA PRÉSENTE INVENTION CONCERNE UN MÉDICAMENT VASO-ACTIF, APPLICABLE EN PARTICULIER AU TRAITEMENT DE L'IMPUISSANCE CHEZ L'HOMME.

De nombreux travaux de recherche dans ce domaine ont permis tout d'abord d'établir le mécanisme et les diverses phases de l'érection, puis de définir les propriétés nécessaires, notamment vaso-actives, de produits qui soient actifs et tolérables. En particulier, des études cliniques multiples ont permis de sélectionner la papavérine et d'en élucider le mode d'action, puis de lui associer des produits alpha-bloquants tels que phénoxybenzamine et phentolamine (BRINDLEY GS - Cavernosal Alphablocate, a new technic for investigating and treating impotence - BRIT. J. PHYCHIATRY 143, 1983). Des travaux antérieurs du Demandeur ont par ailleurs permis d'étudier en détail l'activité isolée et/ou potentialisatrice de nombreuses substances vaso-actives (cf. l'ouvrage "Papavérine et Impuissance" : la voie pharmacologique, Ronald VIRAG, les EDITIONS DU CERI, Paris 1987) notamment l'association des deux alpha-bloquants ci-dessus et de la papavérine.

Parallèlement, l'utilisation clinique de la découverte s'est développée, avec l'utilisation de deux protocoles, à savoir, les perfusions et les auto-injections réalisées par le patient lui-même, juste avant l'acte sexuel (voir l'ouvrage précité).

Cependant, l'étude des premiers résultats observés amène à deux constatations :

1°) que ce soit avec la papavérine seule, avec l'association papavérine et alpha-bloquant ou avec la prostaglandine seule (PGEi), pas plus de 50 % de la totalité des patients impuissants peuvent être traités;

2°) quel que soit le produit, il existe un risque de blocage de l'érection qui varie suivant la drogue utilisée, la dose injectée et l'étiologie de l' impuissance, dans 10 à 30 % des érections obtenues, avec risque d'évolution de cette érection prolongée vers un priapisme véritable.

Compte tenu des données recueillies par les études expérimentales et les résultats cliniques, le produit idéal à injecter serait celui capable de déclencher une érection complète à 100 % des patients avec un risque nul d'érection bloquée et ceci sans manifestation clinique générale : il est apparu qu'un tel produit ne correspond à aucun de ceux employés ou essayés jusqu'à présent.

Compte tenu des conditions locales de survenue de l'érection, le Demandeur a en effet déterminé que le produit idéal pour avoir cette efficacité maximale devrait nécessairement être actif aux diverses phases du cycle érectile, et qu'une telle efficacité ne peut donc être réalisée, dans l'état actuel des produits disponibles, qu'au travers d'une association de plusieurs principes actifs dont l'effet propre de chacun permet de prolonger et de renforcer celui des autres.

Conformément à l'invention, le médicament vaso-actif est constitué par une majeure partie de papavérine, en association avec des principes actifs comprenant au moins un alpha-bloquant et un inhibiteur de la phosphodiastérase, ainsi, avantageusement, qu'une prostaglandine de type PGE1, un agent dopaminergique et un agent atropinique, l'alpha-bloquant étant lui-même constitué par une part alpha$_1$ et une part alpha$_2$, ces constituants repré sentant au total 5 à 20 % en poids par rapport à la papavérine.

On sait que la papavérine agit en tant que myorelaxant de la musculature lisse par action directe sur l'AMP cyclique dont elle empêche l'inactivation. Des actions pharmacologiques secondaires ont été relevées mais n'ont pas été démontrées au niveau du tissu érectile: discrète action alpha-bloquante, action ganglioplégique positive.

Les alpha$_1$-bloquants agissent en inhibant la transmission alpha-adrénergique au niveau post-synaptique ;

Les alpha$_2$-bloquants agissent en inhibant cette transmission au niveau pré-synaptique ;

Le mécanisme local présidant à l'action de la dopamine ou des drogues dopaminergiques sur le tissu érectile n'est pas élucidé à l'heure actuelle mais le Demandeur a constaté cliniquement l'activité positive du produit sur l'érection ;

Le rôle de l'atropine sur le tissu érectile sain est nul. Le produit agit en potentialisation de l'action des autres produits actifs sur le tissu érectile pathologique en interférant sur les mécanismes déclenchant la relaxation du muscle lisse et spécialement de l'EDRF (Endothélium Realesing Factor ou facteur de relaxation d'endothélium) ;

Les inhibiteurs de la phosphodiastérase (type dipyridamole) sont actifs en prolongeant l'action des autres produits et également par leur action anti-aggrégant plaquettaire. Localement ils préviennent le risque de thrombose de sang intracaverneux lorsque l'érection est prolongée ;

La prostaglandine PGE$_1$, ou ses analogues, agit en tant que relaxant de la musculature lisse à un stade préliminaire par rapport à celui de la papavérine dont elle complète l'action, les deux produits se potentialisant.

Il en résulte que chacun des produits actifs du médicament selon l'invention, non seulement a son effet attendu sur un stade défini du mécanisme de l'érection, mais encore, de par cet effet-même, prépare et facilite, voire accentue, l'effet des autres produits actifs, tout en inhibant les effets secondaires nuisibles.

Les exemples suivants, donnés à titre purement illustratif mais nullement limitatif, montrent les effets mentionnés ci-dessus et feront mieux saisir l'intérêt et la portée de l'invention.

Exemple 1 : compositions

Les études cliniques décrites ci-après ont été réalisées à partir des trois compositions qui suivent, par comparaison avec des produits connus.

a) Composition N, comportant pour 1 ml de solution injectable :
- papavérine 20 mg, - tartrate d'infenprodyl 0,75 mg ($\alpha_1$-bloquant)
- atropine 0,025 mg, (anticholinergique)
- yohimbine 0,075 mg ($\alpha_2$-bloquant)
- dipyridamole 0,25 mg, (inhibiteur de la phosphodiastérase)
- piribedil 0,225 mg, (dopaminergique)

b) Composition P comportant, outre les dérivés mentionnés ci-dessus pour la composition M, 33 $\mu$g d'alprostil (Prostaglandine PGE$_1$) par ml de solution injectable;

c) Composition X comportant pour 1 ml de solution injectable :
- papavérine 20 mg,
- phentolamine 1 mg, (alpha-bloquant)
- PGE1 33 $\mu$g, (prostaglandine)
- dipyridamole (0,25 mg, (inhibiteur de phosphodiastérase)

Exemple 2 : essais cliniques

L'étude testant les différents produits entre eux a été menée en injectant 0,2 ml du produit testé pour 100 ml de verge flaccide, volume calculé de façon connue en soi (cf. l'ouvrage précité). Dix minutes après cette injection le patient est soumis à une stimulation sexuelle visuelle (SSV) pendant 10 mn.

Chaque produit a été expérimenté par "randomisation" par série de 23 à 30 patients.

On mesure toutes les trois minutes le volume de la verge au moyen d'un abaque et la rigidité à l'aide d'un appareillage spécifique, le rigidimètre, étalonné de 0 à 200 unités PR (Penrig) dont les valeurs remarquables sont les suivantes :
- jusqu'à 35 PR : pas de variation significative ;
- de 35 à 50 PR : tumescence significative avec importante variation de volume ;
- de 55 à 75 PR : début de rigidité ;
- au-delà de 75 PR : seuil de rigidité nécessaire à la pénétration ;
- à partir de 100 PR : rigidité normale, correspondant à un blocage veineux complet.

On a retenu comme mesure comparative le pourcentage de patients pour lequel une érection complète (rigidité supérieure à 75 PR) est obtenue pendant plus de la moitié du temps de stimulation visuelle et persiste au moins une minute après l'arrêt de cette stimulation.

Les résultats de cette étude sont illustrés par le graphique synoptique annexé, où l'on a représenté des blocs relatifs à chaque produit et dont la hauteur est mesurée par rapport à une échelle graduée de 0 à 100 % d'érection complète. Dans chaque bloc, on a en outre fait figurer le pourcentage d'érection complète non durable après SSV (A) et se prolongeant su moins d'une minute après SSV (B), ainsi que le nombre n de sujets soumis à l'essai. Toutes les mesures ont été effectuées à partir d'une injection locale intra-caverneuse de 0,2 ml de produit, à savoir : un témoin ou placebo de sérum physiologique (1), 40 mg/ml de papavérine (2), 40 mg de papavérine associée à 0,5 mg de phentolamine par ml (3), 33 $\mu$g/ml de PGE$_1$ (4), et compositions M (5), P (6) et X (7) selon l'invention.

En se référant à ce graphique, les résultats démontrent que les trois compositions N, X et P ont produit respectivement 82, 75 et 91 % d'érections complètes suivant les critères choisis. A l'inverse, le placébo, la papavérine, la prostaglandine seule et l'association papavérine/phentolamine ont obtenu respectivement 7, 22, 52 et 54 % d'érections complètes. On peut noter que ces résultats sont obtenus avec une teneur deux fois moindre en papavérine pour les compositions selon l'invention.

Il faut tenir également compte de la capacité d'un produit à provoquer une érection prolongée, cette dernière représentant un risque quand elle se prolonge au-delà de trois heures. Grâce à la présence de l' atropine et de l'inhibiteur de la phosphodiastérase, le pouvoir bloquant des compositions N et P est inférieur à celui des autres associations les plus efficaces à cet égard, à savoir, papavérine, phentolamine, ce qui constitue également un élément supplémentaire favorable vis-à-vis des produits déjà utilisés dans ce domaine.

Exemple 3 : études cliniques

Au total, 264 patients impuissants ont été soumis au traitement par auto-injections avec les compositions selon l'invention.

Après tests préalables, il est pratiqué avant l'acte sexuel une ou deux injections intracaverneuses par semaine. 221 de ces patients (4,12 %) avaient une impuissance organique, 136 d'entre eux (51,51 %) au total auraient constitué sans la médication une indication chirurgicale dont 64 (24 %) étaient des indications à implanter une prothèse pénienne.

Parmi 230 patients régulièrement suivis : 19 sont classés mauvais résultats, 5 sont inclassables par insuffisance d'utilisation de la méthode, 12 sont des résultats moyens (la vie sexuelle ayant été reprise de façon satisfaisante), 176 sont considérés comme bons et 17 comme très bons résultats. Pour 205 de ces 230 patients (soit 89,13 %) la reprise d'une activité sexuelle normale est obtenue. Pour 8,69 % d'entre eux la guérison est totale, le traitement a été interrompu, pour 12,6 % les injections ne sont plus utilisées dans plus de 50 % des actes sexuels et enfin dans 42,6 % des cas le patient est entièrement dépendant du traitement. Ces résultats ont été obtenus sans aucune complication générale ni locale en dehors de 5,8 % d'hématomes au point d'injection, étant entendu que, contrairement aux autres produits injectés, une composition selon l'invention est totalement indolore lorsque le produit pénètre les corps caverneux.

Il est bien entendu que la présente invention n'a été décrite et représentée qu'à titre explicatif mais nullement limitatif et qu'on pourra y apporter toute modification utile, notamment dans le domaine des équivalences techniques, sans sortir de son cadre.

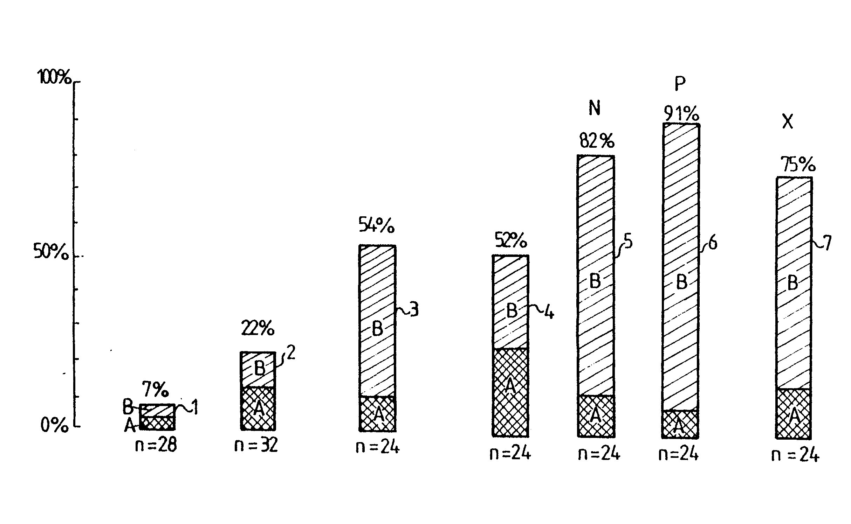

**Revendications**

1. Médicament vaso-actif, applicable au traitement de l'impuissance chez l'homme, du type constitué par une association de principes actifs à la papavérine, caractérisé par le fait qu'il comprend au moins un alpha-bloquant et un inhibiteur de la phosphodiastérase, représentant au total 5 à 20 % en poids par rapport à la papavérine.

2. Médicament selon la revendication 1, caractérisé par le fait qu'il comprend un $alpha_1$-bloquant, un $alpha_2$-bloquant, un anticholinergique, un dopaminergique et l'inhibiteur de la phosphodiastérase, représentant au total 5 à 20 % en poids par rapport à la papavérine.

3. Médicament selon l'une des revendication 1 ou 2, caractérisé par le fait qu'il comprend en outre une prostaglandine de type $PGE_1$ comprise dans les 5 à 20 % en poids par rapport à la papavérine.

4. Médicament selon la revendication 3, caractérisé par le fait qu'il est constitué par la composition : papavérine 20 mg, phentolamine 1 mg, $PGE_1$ 33 μg, dipyridamole 0,25 mg, pour 1 ml de solution injectable.

5. Médicament selon la revendication 2, caractérisé par le fait qu'il est constitué par la composition: papavérine 20 mg, tartrate d'infenprodyl 0,75 mg, atropine 0,025 mg, yohimbine 0,075 mg, dipyridamole 0,25 mg, piribedil 0,225 mg, pour 1 ml de solution injectable.

6. Médicament selon la revendication 5, caractérisé par le fait qu'il comprend en outre 33 μg d'alprostil, pour 1 ml de solution injectable.

EP 0 408 440 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-3 637 157  (BYK GULDEN LOMBERG CHEM. FABRIK GmbH) --- | 1-6 | A 61 K   31/485 A 61 K   31/505 A 61 K   31/557 // (A 61 K   31/485 A 61 K   31:415 A 61 K   31:505) (A 61 K   31/557 A 61 K   31:505 A 61 K   31:485 A 61 K   31:475 A 61 K   31:415) |
| P,A | EP-A-0 346 297  (CENTRO MEDICO HARVEY S.R.L.) ----- | 1-6 | |

**DOMAINES TECHNIQUES RECHERCHES (Int. Cl.5)**

A 61 K

Le présent rapport a été établi pour toutes les revendications

| Lieu de la recherche | Date d'achèvement de la recherche | Examinateur |
|---|---|---|
| LA HAYE | 05-10-1990 | BRINKMANN C. |

EPO FORM 1503 03.82 (P0402)